# FASCICULE DE BREVET EUROPEEN

(11) **EP 0 930 085 B1**
(45) Date de publication et mention de la délivrance du brevet: **10.04.2002**
(21) Numéro de dépôt: 99106100.3
(22) Date de dépôt: 01.08.1994
(51) Int. Cl.: A61N 1/37

(54) **Circuit de protection pour dispositif electronique implantable**
Schutzschaltung für eine implantierbare elektronische Vorrichtung
Protection circuit for an implantable electronic device

(30) Priorité: 11.08.1993 FR 9309853
(43) Date de publication de la demande: 21.07.1999
(62) Demande divisionnaire de: 94401759.9
(73) Titulaire: ELA MEDICAL, F-92541 Montrouge (FR)
(72) Inventeur: Jacobson, Peter, Chanhassen-MN 55317 (US)
(74) Mandataire: Laget, Jean-Loup

(56) Documents cités:
- EP-A- 0 546 666
- US-A- 4 320 763
- US-A- 4 745 923

## Description

L'invention concerne la protection des dispositifs électroniques implantables comprenant des électrodes utilisées pour détection ou stimulation à faible énergie, vis-à-vis d'impulsions de forte énergie. Elle comprend les stimulateurs cardiaques implantables et les défibrillateurs/stimulateurs cardiaques implantables.

Dans le cas présent, le terme défibrillateur est utilisé pour désigner tout dispositif pour éliminer une tachyarythmie par une énergie électrique dépassant sensiblement l'énergie fournie par les stimulateurs cardiaques implantables.

L'invention concerne la protection des circuits reliés aux électrodes à faible énergie, et la protection des tissus du patient sur le site des électrodes à faible énergie, vis-à-vis des propres impulsions de forte énergie du dispositif lui-même, et des impulsions de forte énergie provenant d'autres dispositifs électroniques médicaux tels que des défibrillateurs externes.

L'invention s'applique également à des dispositifs électroniques ayant besoin d'une protection contre des impulsions de forte énergie.

Les stimulateurs cardiaques implantables assurent généralement une protection vis-à-vis d'impulsions externes de forte énergie par une limitation de la tension entre une paire quelconque d'électrodes. Quand un dispositif externe applique une impulsion de forte énergie à cette paire d'électrodes à travers une faible impédance, des courants de forte intensité passent dans les électrodes et le dispositif limiteur. Ces courants de forte intensité peuvent avoir un effet nuisible sur les tissus du patient dans la région des électrodes.

Le brevet US No 4 745 923 au nom de Winstrom décrit un circuit de protection pour un dispositif implantable, relié en série à une sonde d'un stimulateur cardiaque pour le protéger vis-à-vis de fortes tensions et de courants élevés provenant de défibrillateurs et autres sources. Le circuit décrit limite le courant passant dans les deux directions dans une seule sonde (le brevet montre le circuit dans la sonde de retour de la stimulation , ou positive).

Ce circuit utilise une limitation de courant par repli, également fréquemment utilisée dans les alimentations électroniques de puissance du commerce (Section 6,05, Horowitz et Hill, *The Art of Electronics,* Cambridge University Press, 1989). Dans un limiteur de courant par repli, l'impédance reste faible jusqu'à ce que le courant atteigne une première limite préétablie. Quand le courant dépasse cette première limite, l'impédance reste élevée jusqu'à ce que le courant tombe au-dessous d'une seconde limite prédéterminée qui est inférieure à la première.

Le limiteur de Winstrom comporte un parcours de faible impédance entre ses bornes consistant en un transistor à effet de champ (FET) en série avec une résistance de faible valeur. Un transistor bipolaire ouvre le FET quand la tension appliquée à la résistance de faible valeur dépasse le seuil base-émetteur du transistor bipolaire (la première limite préétablie dans le limiteur de courant par repli).

Il est donc clair qu'un dispositif électronique implantable tel qu'un défibrillateur/stimulateur cardiaque doit protéger ses sondes de faible énergie, telles que les sondes de stimulation et de détection, vis-à-vis d'impulsions de défibrillation et autres impulsions de forte énergie. L'art antérieur poursuit cet objectif, mais avec des insuffisances:

Le brevet Winstrom US 4.745.923 fournit un limiteur de courant par repli automatique, pour surmonter les problèmes d'oscillation et de dissipation posés par une limitation à une valeur constante. Cependant, le circuit de Winstrom n'ouvre le commutateur MOSFET dans un parcours à faible impédance qu'après avoir d'abord commandé un second commutateur à transistor bipolaire, dont le retard peut se traduire par une forte pointe de courant dans le limiteur quand un défibrillateur applique une impulsion à front raide dans les sondes de stimulation et de détection. Le brevet US No 4 823 796 au nom de Benson décrit un défibrillateur externe que le circuit de Winstrom pourrait rencontrer. Benson produit une impulsion trapézoïdale par la décharge par transistor d'un condensateur sans inductance en série. Des mesures montrent que ces circuits peuvent produire des fronts de montée de milliers de volts par microseconde. Tout retard dans le circuit de protection se traduit par une pointe de courant qui peut se coupler à un circuit voisin et l'affecter.

Le brevet Winstrom US 4.745.923 décrit seulement un limiteur bidirectionnel qui doit être utilisé dans une sonde unique. L'idée est de limiter le courant dans une sonde d'une paire de sondes quand un défibrillateur applique l'énergie selon l'une ou l'autre polarité. Dans des défibrillateurs/stimulateurs cardiaques à deux chambres, avec deux paires de sondes, le circuit de Winstrom aurait besoin d'être appliqué à trois des quatre conducteurs. Ceci se traduit par un nombre de composants plus élevé que dans le cas d'un limiteur unidirectionnel dans chaque sonde.

Le but de la présente invention est donc de fournir une protection améliorée pour les sondes de stimulation et de détection à faible énergie de dispositifs médicaux électroniques, l'amélioration consistant en:
1. La protection vis-à-vis d'impulsions externes de forte énergie de l'une ou l'autre polarité.
2. La limitation du courant passant par les sondes de stimulation et de détection au lieu de la limitation de la tension entre elles, pour protéger les tissus cardiaques dans la région des électrodes de stimulation et de détection.
3. L'activation automatique du limiteur, pour fournir une protection vis-à-vis d'impulsions externes de forte énergie, de même que des impulsions de forte énergie fournies par le dispositif lui-même.
4. Une limitation du courant par repli pour réduire la dissipation de puissance et éliminer les oscillations dans le limiteur.
5. L'activation du limiteur de courant par repli par un unique commutateur à transistor pour fournir une réponse rapide à des impulsions de défibrillation externes à front raide.
6. L'utilisation d'un unique limiteur de courant unidirectionnel dans chaque sonde de stimulation et de détection.

Dans son mode de réalisation préféré, l'invention prévoit un limiteur automatique unidirectionnel de courant par repli dans chaque sonde de stimulation et de détection, qui limite les courants pénétrant dans les sondes. En présence d'une impulsion externe de forte énergie, seul un courant limité peut pénétrer dans chaque sonde de stimulation et de détection, et ainsi seul un courant limité peut passer dans l'une quelconque des électrodes de stimulation et de détection.

Chaque limiteur automatique de courant par repli limite rapidement le courant à une première valeur au moyen d'un unique commutateur à transistor, et un peu plus tard ouvre entièrement ce premier commutateur avec un second commutateur jusqu'à ce que le courant tombe au-dessous d'une seconde valeur plus basse.

L'invention a pour objet un dispositif de limitation du courant circulant dans une direction, d'une entrée non protégée vers une sortie protégée, utilisable en série avec la sonde d'un appareil médical implanté ayant des électrodes de stimulation à faible énergie et de détection selon la revendication 1. L'invention comportant
. une source délivrant une tension de polarisation par l'intermédiaire d'une résistance de polarisation ;
. un premier commutateur ayant une condition de circuit ouvert, une condition de limitation de courant, et une condition de circuit fermé, le premier commutateur ayant une entrée reliée à l'entrée non protégée et une sortie ;
. une résistance de faible valeur reliée à la sortie du premier commutateur, ayant une tension en réponse au passage du courant à travers le premier commutateur ;
. un deuxième commutateur ayant une condition de circuit ouvert et une condition de circuit fermé ;
et qui comporte :
. un diviseur de tension relié à l'entrée non protégée et à la sortie protégée et fournissant une tension de contrôle correspondant à la tension entre l'entrée non protégée et la sortie protégée ; de sorte que le premier commutateur soit polarisé dans la condition de circuit fermé lorsque la tension de ladite résistance de faible valeur est inférieure d'une première quantité prédéterminée à la tension de polarisation, et passe automatiquement à la condition de limitation lorsque la tension de ladite résistance de faible valeur n'est pas inférieure de ladite première quantité prédéterminée à la tension de polarisation, et que le deuxième commutateur change automatiquement de condition entre la condition de circuit ouvert lorsque la tension de contrôle est inférieure à une deuxième quantité prédéterminée, et la condition de circuit fermé lorsque la tension de contrôle est supérieure à la deuxième quantité prédéterminée, la condition de circuit fermé abaissant effectivement la tension de polarisation pour maintenir le premier commutateur dans la condition de circuit ouvert.

Selon l'invention :
- le premier commutateur comporte un FET ayant une gâchette reliée à la tension de polarisation, un drain relié à l'entrée non protégée, et une source reliée à ladite résistance de faible valeur, et la première tension prédéterminée est la différence entre la tension de polarisation et la tension de seuil du FET ;
- le deuxième commutateur est constitué par un transistor bipolaire ayant un collecteur relié à la résistance de polarisation, un émetteur relié à la sortie protégée et une base reliée à la tension de contrôle du diviseur de tension, la deuxième tension prédéterminée étant la tension de seuil entre base et émetteur du transistor ;
- le diviseur de tension comporte une première résistance branchée entre l'entrée non protégée et la base du transistor, et une deuxième résistance branchée entre la base du transistor et la sortie protégée ;
- une diode Zener est branchée entre le collecteur et l'émetteur du transistor.

L'invention a encore pour objet un appareil médical comprenant le dispositif de protection ci-dessus, caractérisé en ce que l'appareil médical est un stimulateur cardiaque ayant une pluralité de sondes reliées au tissu cardiaque, de sorte qu'un circuit unidirectionnel de limitation de courant est interposé en série avec chaque sonde, avec sa sortie protégée reliée au stimulateur cardiaque.
La figure 1 est une vue d'ensemble de l'invention appliquée à un défibrillateur/stimulateur cardiaque.
La figure 2 est un diagramme schématique d'un limiteur unidirectionnel de courant par repli.

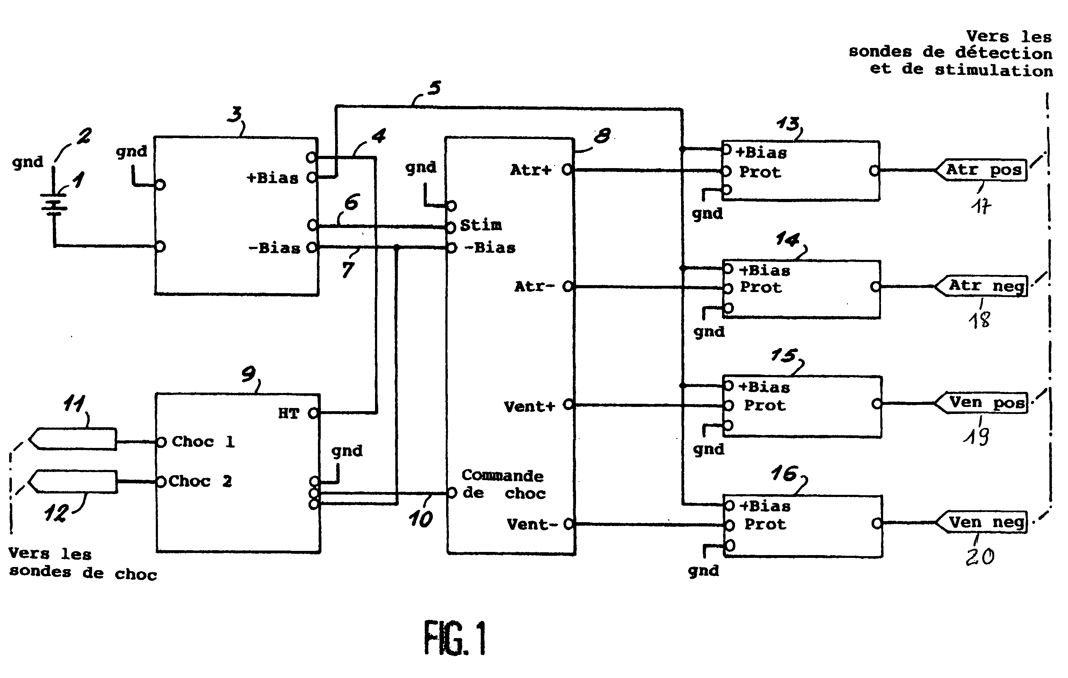

La figure 1 est une vue d'ensemble de l'invention appliquée à un défibrillateur/stimulateur cardiaque, avec des circuits de stimulation et de détection auriculaires et ventriculaires, et avec des chocs multiphasés. La pile 1 alimente le dispositif. La masse 2 sert de tension de référence (0 volts) pour tous les signaux du dispositif, sauf indication contraire. Une alimentation de puissance 3 convertit la tension de la pile en d'autres tensions pour alimenter le reste du dispositif:
* haute tension en 4 pour le générateur de chocs, approximativement 0,75 KV. Le dispositif est couplé à la polarisation négative et non à la masse.
* polarisation positive en 5, pour les limiteurs, d'approximativement 9,0V.
* tension de stimulation en 6, d'approximativement -6,0 V.
* polarisation négative en 7 pour les circuits basse tension et le générateur de chocs, d'approximativement -9,0 V.

En se référant toujours à la figure 1, les circuits basse tension 8 fournissent la stimulation, la détection et la commande. Ils fonctionnent entre la masse 2 et la polarisation négative 7. Comme la valeur de la tension de stimulation en 6 est comprise entre ces deux tensions d'alimentation, ces circuits basse tension peuvent alors générer et mesurer les signaux de stimulation et de détection directement sans décalage de niveau. Quand les circuits basse tension détectent des signaux auriculaires et/ou ventriculaires qui nécessitent un choc, ils le signalent au générateur de chocs 9 par le bus de commande de choc 10.

Toujours à la figure 1, le générateur de chocs 9 envoie des chocs à haute tension entre les sondes de chocs 11 et 12, quand les circuits basse tension le déclenchent par l'intermédiaire du bus de commande de chocs 10. L'énergie de choc provient de la haute tension 4 avec référence à la polarisation négative 7.

Finalement, à la figure 1, les limiteurs 13 à 16 protègent les circuits basse tension 8, et le tissu adjacent aux électrodes de stimulation et de détection reliées aux sondes 17 à 20, vis-à-vis des chocs appliqués par le générateur de chocs 9 ou par un défibrillateur externe.

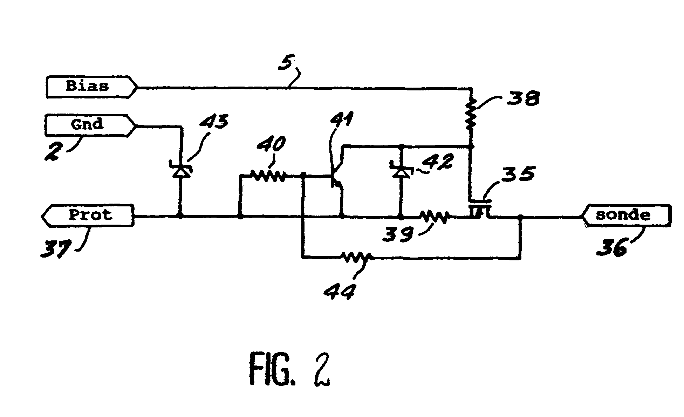

En se référant maintenant à la figure 2, les circuits limiteurs, représentés à la figure 1 en 13 à 16, comprennent chacun un commutateur à transistor haute tension 35, de préférence un MOSFET à canal N. Ces transistors existent dans le commerce avec petite surface de silicium, avec des seuils d'approximativement 5 Volts et des résistances en conduction qui sont typiquement inférieures à 10 Ohms pour une tension entre gâchette et source de 9 Volts; par exemple, les types industriels IRFBG20 ou BUK456/1000B.

A la figure 2, les tensions à la borne 36 de la sonde et à la borne 37 de protection du limiteur, pendant le fonctionnement normal et en l'absence d'impulsions internes ou externes de haute énergie, sont comprises entre celle de l'alimentation de polarisation négative et quelques centaines de millivolts au-dessus de la masse.

Alors que les tensions en 36 et 37 restent dans cette plage de fonctionnement normal, la tension de polarisation positive en 5 maintient le transistor 35 à l'état passant, par l'intermédiaire de la résistance de polarisation 38. Comme le transistor 35 ne tire pas de courant de gâchette à l'exception du courant de fuite, l'alimentation de polarisation positive n'a besoin que d'un faible courant. Comme l'alimentation doit générer l'alimentation de polarisation positive en inversant la tension de la pile, cette faible demande de courant permet d'utiliser un petit inverseur capacitif avec peu de composants, et éventuellement intégrable. Si par contre l'architecture de l'invention plaçait la pile au-dessus de la masse et inversait la tension de la pile pour obtenir la stimulation, ceci exigerait une capacité de courant beaucoup plus élevée dans l'inverseur (notamment pour une stimulation rapide pour une réversion d'arythmie), éliminant la possibilité d'une intégration.

Le circuit de la figure 2 comprend une résistance de faible valeur 39 en série avec la source du transistor 35. La résistance constitue avec le transistor 35 un parcours de basse impédance entre la sonde 36 et la connexion protégée 37 quand le transistor 35 est passant à l'état normal dans une première condition de circuit fermé ne limitant pas le courant entre l'entrée 36 et la sortie 37. Si le courant à travers la résistance 39 augmente de manière que la tension aux bornes de cette résistance dépasse le seuil du transistor 35, ceci limite le courant dans le transistor 35 à sa tension de seuil divisée par la valeur de la résistance 39, soit typiquement 0,15 A. Le transistor 35 est alors dans une deuxième condition de limitation du courant.

Quand le transistor 35 limite ainsi le courant, la tension à la borne 36 de la sonde monte jusqu'à ce que la tension base-émetteur du transistor 41, fixée par un diviseur de tension consistant en des résistances 44 et 40, dépasse le seuil de ce transistor 41. Ceci rend le transistor 41 passant, limitant la tension gâchette-source du transistor 35 à zéro. Ceci bloque le transistor 35 et il ne dissipe plus de puissance. Il est alors dans une troisième condition de circuit ouvert, et entre l'entrée 36 et la sortie 37, se trouve la résistance élevée et fixe 44-40. Le circuit reste dans cet état d'impédance élevée jusqu'à ce que la tension à ses bornes tombe à un point où le transistor 41 est bloqué, permettant à la résistance de polarisation 38 de rendre à nouveau le transistor 35 passant, rétablissant le parcours à faible impédance.

Les diodes Zener 42 et 43 de la figure 2 protègent la jonction gâchette-source du transistor 35, et le circuit basse tension 8 de la figure 1, respectivement.

Bien que l'invention ait été décrite en référence à un mode de réalisation particulier, on comprendra que ce mode de réalisation soit simplement illustratif de l'application des principes de l'invention. De nombreuses autres modifications peuvent être faites et d'autres agencements peuvent être envisagés sans s'écarter du champ des revendications ci-jointes.

## Revendications

1. Dispositif de limitation du courant circulant dans une direction, d'une entrée non protégée (36) vers une sortie protégée (37), utilisable en série avec la sonde d'un appareil médical implanté ayant des électrodes de stimulation à faible énergie et de détection comportant :
- une source délivrant une tension de polarisation (5) par l'intermédiaire d'une résistance de polarisation (38) ;
- un premier commutateur (35) ayant une condition de circuit ouvert et une condition de circuit fermé, le premier commutateur comportant un FET ayant une gâchette reliée à la tension de polarisation (5), un drain relié à l'entrée non protégée (36), et une source reliée à une résistance de faible valeur (39),
- ladite résistance de faible valeur (39) ayant une tension en réponse au passage du courant à travers le premier commutateur (35) ;
- un deuxième commutateur (41) ayant une condition de circuit ouvert et une condition de circuit fermé, le deuxième commutateur (41) étant constitué par un transistor bipolaire ayant un collecteur relié à la résistance de polarisation (38), un émetteur relié à la sortie protégée (37) et une base reliée à la tension de contrôle d'un diviseur de tension (44, 40);
- ledit diviseur de tension (44, 40) comportant une première résistance (44) branchée entre l'entrée non protégée (36) et la base du transistor (41), et une deuxième résistance (40) dont une extrémité est reliée à la base du transistor bipolaire (41)
**caractérisé en ce que** l'autre extrémité de ladite deuxième résistance (40) est reliée à la sortie protégée (37), ledit diviseur de tension fournissant une tension de contrôle correspondant à la tension entre l'entrée non protégée (36) et la sortie protégée (37) ; de sorte que le premier commutateur (35) soit polarisé dans la condition de circuit fermé lorsque la tension de ladite résistance de faible valeur (39) est inférieure d'une première tension prédéterminée à la tension de polarisation, et passe automatiquement à une condition de limitation lorsque la tension de ladite résistance de faible valeur (39) n'est pas inférieure de ladite première tension prédéterminée à la tension de polarisation, et que le deuxième commutateur (41) change automatiquement de condition entre la condition de circuit ouvert lorsque la tension de contrôle est inférieure à une deuxième tension prédéterminée, et la condition de circuit fermé lorsque la tension de contrôle est supérieure à la deuxième tension prédéterminée, la condition de circuit fermé abaissant effectivement la tension de polarisation pour maintenir le premier commutateur (35) dans la condition de circuit ouvert.

2. Dispositif selon la revendication 1, **caractérisé en ce que** la première tension prédéterminée est la différence entre la tension de polarisation (5) et la tension de seuil du FET.

3. Dispositif selon la revendication 1, **caractérisé en ce que** la deuxième tension prédéterminée est la tension de seuil entre base et émetteur du transistor (41).

4. Dispositif selon la revendication 1, **caractérisé en outre, en ce qu'**une diode Zener (42) est branchée entre le collecteur et l'émetteur du transistor (41).

5. Appareil médical comprenant le dispositif de protection selon l'une des revendications 1 à 4, **caractérisé en ce que** l'appareil médical est un stimulateur cardiaque ayant une pluralité de sondes reliées au tissu cardiaque, de sorte qu'un circuit unidirectionnel de limitation de courant est interposé en série avec chaque sonde, avec sa sortie protégée reliée au stimulateur cardiaque.

## Claims

1. Limiting device for unidirectionnal current circulating from an unprotected input (36) to a protected output (37), for use in series with the lead of an implanted medical apparatus having low energy stimulation and detection electrodes comprising :
- a source delivering a bias voltage (5) through a bias resistor (38) ;
- a first switch (35) having an open circuit condition and a closed circuit condition, the first switch comprising a FET having a gate connected to the bias voltage (5), a drain connected to the unprotected input (36) and a source connected to a low value resistor (39) ;
- said low value resistor (39) having a voltage in response to the current flow through the first switch (35);
- a second switch (41) having an open circuit condition and a closed circuit condition, the second switch (41) consisting of a bipolar transistor having a collector connected to the bias resistor (38), an emitter connected to the protected output (37) and a base connected to the control voltage of a voltage divider (44, 40);
- said voltage divider (44, 40) comprising a first resistor (44) connected between the unprotected input (36) and the transistor base (41), and a second resistor (10) one end of which is connected to the base of the bipolar resistor (41)
**characterized in that** the other end of said second resistor (40) is connected to the protected output (37), said voltage divider giving a control voltage corresponding to the voltage between the unprotected input (36) and the protected output (37) ; in such a way that the first switch (35) is polarized in the closed circuit condition when the voltage of said low value resistor (39) is less than the bias voltage by a first predetermined voltage and automatically changes to a limiting condition when the voltage of said low value resistor (39) is not less than the bias voltage by said first predetermined voltage, and that the second switch (41) automatically changes of condition between the open circuit condition when the control voltage is less than a second predetermined voltage, and the closed circuit condition when the control voltage is greater than the second predetermined voltage, the closed circuit condition effectively lowering the bias voltage to maintain the first switch (35) in the open circuit condition.

2. Device according to claim 1,
**characterized in that** the first predetermined voltage is the difference between the bias voltage (5) and the FET threshold voltage.

3. Device according to claim 1,
**characterized in that** the second predetermined voltage is the threshold voltage between the transistor (41) base and emitter.

4. Device according to claim 1,
characterized further in that a Zener diode (42) is connected between the transistor (41) collector and emitter.

5. Medical apparatus comprising the protection device according to one of the claims 1 to 4,
**characterized in that** the medical apparatus is a cardiac pacemaker having a plurality of leads connected to the cardiac tissue in such a way that a limiting unidirectionnal current circuit is interposed in series with each lead, with its protected output connected to the cardiac pacemaker.

## Patentansprüche

1. Vorrichtung zur Begrenzung des in eine Richtung, von einem nicht geschützten Eingang (36) zu einem geschützten Ausgang (37), zirkulierenden Stroms, die in Reihe zu der Sonde eines implantierten medizinischen Geräts mit Niedrigenergie-Stimulierungselektroden und Erfassungselektroden benutzbar ist, mit:
- einer Source, die eine Polarisierungsspannung (5) Über einen Polarisierungswiderstand (38) liefert;
- einem ersten Schalter (35), der einen offenen Schaltzustand und einen geschlossenen Schaltzustand aufweist, wobei der erste Schalter einen Feldeffekt-(FET-)Transistor mit einem mit der Polarisierungsspannung (5) verbundenen Gate, einem mit dem nicht geschützten Eingang (36) verbundenen Drain und einer mit einem niedrigwertigen Widerstand (39) verbundenen Source umfasst,
- wobei der niedrigwertige Widerstand (39) eine Spannung im Ansprechen auf das Fließen des Stroms über den ersten Schalter (35) aufweist;
- einem zweiten Schalter (41), der einen offenen Schaltzustand und einen geschlossenen Schaltzustand aufweist, wobei der zweite Schalter (41) durch einen bipolaren Transistor mit einem mit dem Polarisierungswiderstand (38) verbundenen Kollektor, einem mit dem geschützten Ausgang (37) verbundenen Emitter und einer mit der Steuerspannung eines Spannungsteilers (44, 40) verbundenen Basis gebildet ist;
- wobei der Spannungsteiler (44, 40) einen ersten Widerstand (44) umfasst, der zwischen dem nicht geschützten Eingang (36) und der Basis des Transistors (41) angeschlossen ist, sowie einen zweiten Widerstand (40) umfaßt, dessen eines Ende mit der Basis des bipolaren Transistors (41) verbunden ist,
**dadurch gekennzeichnet, dass**
das andere Ende des zweiten Widerstands (40) mit dem geschützten Ausgang (37) verbunden ist, wobei der Spannungsteiler eine Steuerspannung liefert, die der Spannung zwischen dem nicht geschützten Eingang (36) und dem geschützten Ausgang (37) entspricht; so dass der erste Schalter (35) in geschlossenem Schaltzustand polarisiert ist, wenn die Spannung des niedrigwertigen Widerstands (39) geringer ist als eine erste, für die Polarisierungsspannung vorbestimmte Spannung, und automatisch in einen Begrenzungszustand übergeht, wenn die Spannung des niedrigwertigen Widerstands (39) nicht geringer als die erste, für die Polarisierungsspannung vorbestimmte Spannung ist, und dass der zweite Schalter (41) automatisch den Zustand ändert zwischen dem offenen Schaltzustand, wenn die Steuerspannung geringer ist als eine vorbestimmte zweite Spannung, und dem geschlossenen Schaltzustand, wenn die Steuerspannung größer ist als die zweite vorbestimmte Spannung, wobei der geschlossene Schaltzustand die Polarisierungsspannung wirksam verringert, um den ersten Schalter (35) im offenen Schaltzustand zu halten.

2. Vorrichtung nach Anspruch 1, **dadurch gekennzeichnet, dass** die erste vorbestimmte Spannung die Differenz zwischen der Polarisierungsspannung (5) und der Schwellenspannung des FET ist.

3. Vorrichtung nach Anspruch 1, **dadurch gekennzeichnet, dass** die zweite vorbestimmte Spannung die Schwellenspannung zwischen Basis und Emitter des Transistors (41) ist.

4. Vorrichtung nach Anspruch 1, im weiteren **dadurch gekennzeichnet, dass** eine Zener-Diode (42) zwischen dem Kollektor und dem Emitter des Transistors (41) angeschlossen ist.

5. Medizinisches Gerät mit der Schutzvorrichtung gemäß einem der Ansprüche 1 bis 4, **dadurch gekennzeichnet, dass** das medizinische Gerät ein Herzschrittmacher mit einer Vielzahl von mit dem Herzgewebe verbundenen Sonden ist, so dass eine unidirektionale Schaltung zur Strombegrenzung in Reihe zu jeder Sonde geschaltet ist, wobei ihr geschützter Ausgang mit dem Herzschrittmacher verbunden ist.
